# FASCICULE DE BREVET EUROPEEN

(11) **EP 0 773 806 B1**
(45) Date de publication et mention de la délivrance du brevet: **10.06.1998**
(21) Numéro de dépôt: 94906248.3
(22) Date de dépôt: 07.02.1994
(51) Int. Cl.: A61M 5/315, A61M 5/24

(54) **INSTRUMENT DOSEUR, NOTAMMENT D'INJECTION DE LIQUIDE MEDICAMENTEUX**
DOSIERVORRICHTUNG, INSBESONDERE ZUM EINSPRITZEN VON MEDIKAMENTEN
METERING INSTRUMENT, PARTICULARLY FOR INJECTING MEDICINAL LIQUIDS

(30) Priorité: 08.02.1993 FR 9301557
(43) Date de publication de la demande: 21.05.1997
(73) Titulaire: LABORATOIRE AGUETTANT, 69007 Lyon (FR)
(72) Inventeur: FREZZA, Pierre, F-69390 Vourles (FR)
(74) Mandataire: Bratel, Gérard
(86) Numéro de dépôt international: FR9400137
(87) Numéro de publication internationale: WO9417846

(56) Documents cités:
- EP-A- 0 037 696
- EP-A- 0 416 975
- EP-A- 0 496 141
- EP-A- 0 498 737
- WO-A-91/10460

## Description

La présente invention a pour objet un instrument doseur, notamment de liquide médicamenteux, permettant de dispenser successivement plusieurs doses d'un liquide ou autre fluide, à partir d'une charge ou recharge.

Cet instrument vise de manière non exclusive un instrument d'injection de type seringue, permettant d'administrer ou de s'administrer par voie parentérale, plusieurs doses d'un liquide médicamenteux, prélevées dans une ampoule, telle qu'une ampoule de type carpule.

Le document FR-A-1 170 312, concerne un dispositif d'injection par seringue, dans lequel la tige de piston est actionnée par une crémaillère, cette crémaillère étant entraînée dans le sens de l'injection par un premier dispositif à cliquet, et étant retenue dans le sens de déplacement inverse par un second dispositif à cliquet.

Le document EP-A-0 416 975, concerne un dispositif permettant plusieurs injections à partir d'une même recharge, comprenant un corps tubulaire destiné à être maintenu par l'utilisateur, un réservoir contenant la charge de liquide, disposée à une extrémité du corps, un poussoir monté coaxialement et libre en translation à l'intérieur du corps, actionnable par l'utilisateur par une de ses extrémités qui dépasse du corps du coté opposé au réservoir, une queue en forme de crémaillère hélicoïdale, montée à l'intérieur du poussoir, comprenant une extrémité en appui contre le fond du réservoir. Un premier moyen d'encliquetage, associé au corps, permet le déplacement de la queue dans le sens de l'injection, mais non dans le sens inverse, tandis qu'un second moyen d'encliquetage associé au poussoir se bloque sur la queue lorsque celui-ci est déplacée dans le sens d'injection, et échappe à la queue lorsque le poussoir est déplacé dans l'autre sens.

Le volume de la dose injectée est déterminé essentiellement par le nombre de déclics correspondant au passage des crans de la queue le long des moyens d'encliquetage associés au corps. Cette solution n'est absolument pas satisfaisante, dans la mesure où elle ne présente aucune sécurité, notamment lorsque la personne devant s'effectuer une injection est une personne handicapée, ou ayant perdu ses facultés de manipulation, ou encore, en cas d'urgence, où il est nécessaire d'injecter très rapidement une dose prédéterminée d'un principe actif.

Une autre solution consiste à disposer d'un mécanisme de réglage de la position initiale du poussoir fixant la course en translation de ce dernier par rapport à la queue, ce réglage en translation étant réalisé par l'intermédiaire d'un filetage extérieur hélicoïdal que comporte la queue, sur lequel est engagé un cran intérieur prévu sur le poussoir, la rotation du poussoir par rapport à la queue permettant de le rentrer à l'intérieur du corps ou de le sortir de celui-ci selon une course réglable. Cette modification de la position axiale de départ du poussoir détermine la course de celui-ci vis-à-vis du corps et, par suite, le volume de la dose d'injection.

Ce dernier dispositif comporte un grand nombre de pièces ne pouvant être assemblées les unes aux autres de façon automatique, ce qui se traduit par un coût de revient élevé, incompatible avec un usage unique. Dans ces conditions un tel instrument ne peut être jetable.

Par ailleurs, son mode d'utilisation demeure compliqué et ne peut, de même que le dispositif décrit précédemment, être mis en oeuvre par des personnes handicapés, ne disposant pas de toutes leurs facultés, ou en cas d'urgence où une dose doit être injectée très rapidement.

Le but de l'invention est de fournir un instrument doseur, pour injection de liquide médicamenteux, de conception simple, réalisé avec un faible nombre de pièces, assemblables très rapidement et de façon automatique, ergonomique, offrant une grande précision du volume de la dose dispensée ou injectée, avec possibilité de réglage de plusieurs volumes différents de doses.

A cet effet, l'instrument qu'elle concerne, du type comprenant :
- un corps tubulaire, destiné à être maintenu par l'utilisateur,
- un réservoir contenant la charge ou recharge du liquide, rapporté sur ou intégré dans ledit corps, ce réservoir comprenant une paroi tubulaire dans l'axe du corps, un piston formant joint d'étanchéité, fermant une extrémité de ladite paroi tubulaire, déplaçable vers l'autre extrémité, dans un sens dit de référence, et un bouchon perforable fermant l'autre extrémité, par lequel s'effectue le passage d'une dose du liquide,
- un poussoir tubulaire monté coaxialement et libre en translation à l'intérieur du corps tubulaire, actionnable par l'utilisateur par une de ses extrémités qui dépasse du corps,
- une queue, formant crémaillère, montée à l'intérieur et dans l'axe du poussoir, comprenant une extrémité en appui contre le piston du réservoir,
- un premier moyen d'encliquetage disposé sur le corps, échappant à la queue dans le sens de référence, et bloquant la queue dans l'autre sens, et
- un deuxième moyen d'encliquetage disposé sur le poussoir, se bloquant sur la queue dans le sens de référence, et échappant à ladite queue dans l'autre sens,
la queue cylindrique comportant une pluralité de crans extérieurs successifs, ménagés selon l'axe du corps, sur une longueur au moins égale à la course du piston nécessaire pour vider le réservoir, coopérant à la fois avec le premier moyen d'encliquetage et le deuxième moyen d'encliquetage, caractérisé en ce que les crans extérieurs de la queue sont annulaires,
en ce que le corps présente plusieurs fentes axiales de longueurs différentes les unes des autres, réparties sur la périphérie du corps,
et en ce que le poussoir comporte un ergot qui, faisant saillie de sa face extérieure, est destiné à être engagé dans l'une des fentes du corps, dont la longueur correspond à la course choisie pour le poussoir.

Les moyens d'encliquetage du corps et du poussoir sur la crémaillère de la queue sont constitués par des séries de dents venant de moulage, respectivement, avec le corps et avec le poussoir, chaque dent étant adaptée à une pénétration entre deux crans successifs de la crémaillère. Le nombre de pièces constitutives de cet instrument est donc réduit, puisqu'il comprend essentiellement le corps, le poussoir et la queue. Le corps est une pièce monobloc assurant les fonctions de dosage, grâce aux différentes fentes qu'il comporte, de cliquets anti-retour, et d'affichage de la dose, indiqué en regard de chaque fente.

Le poussoir cumule pour sa part les fonctions d'armement, d'injection, de repérage de la dose et de réglage de la dose, ainsi que de cliquets anti-retour par la possibilité qu'il a d'exercer une pression sur la queue lors de l'enfoncement du piston, et de coulisser sur celle-ci lors d'un déplacement en sens inverse.

La queue formant crémaillère cumule les fonctions d'entraînement du piston, d'anti-retour et de dosage, l'unité minimale d'injection étant égale à la longueur d'un cran.

Le corps et le poussoir étant tubulaires et ouverts à leurs extrémités, le montage de l'un dans l'autre est effectué par simple emboîtement, de même que la mise en place de la queue est réalisée par simple engagement à l'intérieur du poussoir, depuis l'extérieur, vers l'extrémité du poussoir située du côté du réservoir. Ce montage est extrêmement simple et peut être automatisé.

Il est important de noter que, grâce à la section circulaire de chaque cran de la crémaillère, il est possible de faire pivoter le poussoir autour de la queue, et à l'intérieur du corps, pour faire passer l'ergot solidaire du poussoir d'une fente à l'autre du corps, pour changer le volume de la dose.

Avantageusement, la longueur de chaque fente axiale ménagée dans le corps permet une course de l'ergot égale à un nombre entier multiplié par la longueur d'un cran de la crémaillère ménagée sur la queue.

Suivant une autre caractéristique de l'invention, l'ergot faisant saillie de la face extérieure du poussoir est agencé pour s'effacer radialement lorsque le poussoir est entraîné en rotation dans un sens à l'intérieur du corps, et/ou axialement lors de l'introduction du poussoir à l'intérieur du corps.

A cet effet, l'ergot faisant saillie de la face extérieure du poussoir est constitué par une nervure axiale faisant saillie vers l'extérieur, ménagée à l'extrémité libre d'une languette délimitée dans la paroi du poussoir par une fente axiale et deux fentes circonférentielles s'étendant sur une partie de la périphérie du poussoir.

Lorsque le poussoir est entraîné en rotation à l'intérieur du corps, dans un sens de rotation, la languette portant l'ergot tend à basculer vers l'intérieur, permettant l'escamotage de l'ergot le long de la paroi interne du corps.

En outre, afin de faciliter l'introduction du poussoir à l'intérieur du corps, le bord de la nervure formant ergot, situé en avant dans le sens d'introduction du poussoir dans le corps est arrondi, tandis que le bord arrière de cette nervure est droit et perpendiculaire à l'axe du poussoir.

Selon une autre caractéristique de l'invention, l'extrémité du poussoir extérieure au corps et actionnable par l'utilisateur est équipée d'une collerette destinée à venir prendre appui contre le bord arrière du corps, en fin de course d'injection.

Cette collerette permet de tirer et pousser le poussoir, pour réaliser respectivement l'armement et l'injection, ainsi que de le tourner pour adapter l'injecteur au volume de la dose à délivrer.

Avantageusement, les différentes fentes, ménagées dans le corps, présentent des extrémités avant situées dans un même plan perpendiculaire à l'axe du corps, la distance entre ce plan et l'extrémité arrière du corps étant légèrement supérieure à la distance entre le bord avant de l'ergot ménagé sur le poussoir et la collerette disposée à l'extrémité arrière de celui-ci.

Il résulte de cette caractéristique que, lors de la fin de course vers l'avant du poussoir, la collerette de celui-ci prend appui contre le bord supérieur du corps, avant que l'ergot arrive en butée à l'extrémité inférieure de la fente dans laquelle il est monté déplacable. Cela garantit une butée de fin de course franche, et par suite l'exactitude du volume injecté.

Pour faciliter le repérage du volume dosé, la collerette du poussoir est équipée d'un doigt d'indexation aligné avec l'ergot et orienté en direction de l'avant, ce doigt se trouvant en regard d'un marquage que comporte le corps dans l'alignement de chaque fente. A son extrémité supérieure, la queue est équipée d'une collerette de diamètre extérieur inférieur au diamètre intérieur du poussoir, mais ne permettant pas le passage du moyen d'encliquetage du poussoir.

Ainsi , l'opérateur est immédiatement prévenu lorsque le volume de liquide disponible demeurant dans le réservoir, est inférieur au volume de la dose devant être injectée. En outre, il n'est pas possible, après retrait du réservoir, de sortir la queue comportant la crémaillère par l'avant, de telle sorte que l'instrument n'est pas réutilisable.

Une forme d'exécution de cet instrument est décrite, ci-après, en référence au dessin schématique annexé dans lequel :
Figure 1 est une vue de face de cet instrument d'injection du type stylo-injecteur avant première utilisation, le réservoir étant plein
Figure 2 en est une vue en coupe transversale et à échelle agrandie selon la ligne II-II de figure 1 ;
Figure 3 en est une vue en coupe longitudinale, selon la ligne III-III de figure 2, après retrait du capuchon ;
Figures 4 et 5 sont deux vues en coupe longitudinale similaires à figure 3, au cours de deux phases d'utilisation ;
Figure 6 est une vue en coupe longitudinale de l'instrument, à l'exception du poussoir qui est représenté en vue de côté.

L'instrument représenté au dessin comprend un corps 2, tubulaire, de section circulaire, en matière synthétique, présentant à proximité de son extrémité avant un filetage intérieur 3 servant au vissage d'une enveloppe 4 contenant un réservoir 5, ou carpule, pour le liquide médicamenteux 6 à injecter. La carpule 5 est fermée à son extrémité postérieure par un piston 7. Ce piston est déplacable dans un sens dit sens de référence 8 correspondant au sens de vidage de la carpule. Un autre bouchon 9 ferme l'autre extrémité de la carpule, qui est traversé par une aiguille d'injection 10. L'enveloppe 4 comporte une fente longitudinale 12 permettant de visualiser le niveau de liquide à l'intérieur de la carpule.

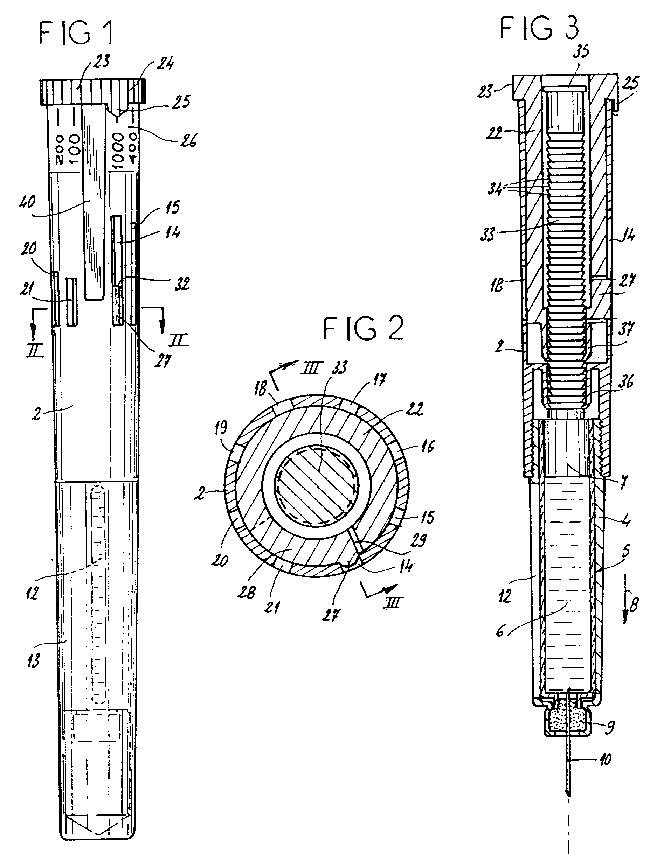

Comme montré à la figure 1, un capuchon amovible 13 coiffe l'extrémité avant de l'instrument, avec pour fonction essentielle de protéger l'aiguille 10 entre deux utilisations successives de l'instrument.

Comme montré notamment aux figures 1 et 2, le corps comporte un certain nombre de fentes longitudinales, huit dans l'exemple représenté, référencées 14-21.

L'instrument comprend également un poussoir tubulaire 22 de section circulaire, ouvert à ses deux extrémités, constitué par une pièce en matière synthétique moulée, destinée à pouvoir coulisser et pivoter à l'intérieur du corps 2.

Ce poussoir 22 comprend, à son extrémité supérieure, c'est-à-dire à son extrémité opposée à celle située du côté du réservoir 5, une collerette 23 destinée à l'armement et à l'injection d'une dose. Cette collerette 23 comporte, pour des raisons de facilité de manipulation, un crantage extérieur 24, et possède un index 25, tourné du côté de l'avant de l'instrument, susceptible de venir en position de recouvrement de la face extérieure du corps, en fin d'injection, et coopérant avec des marques 26 ménagées sur le corps pour visualiser le volume de la dose injectée ou à injecter.

En regard du doigt 25, le poussoir 22 comporte un ergot 27 faisant saillie vers l'extérieur, destiné à être engagé dans une fente 14-21 du corps. L'ergot 27 est constitué par une nervure axiale faisant saillie vers l'extérieur, ménagée à l'extrémité libre d'une languette 28, délimitée dans la paroi du poussoir par une fente axiale 29, et par deux fentes circonférentielles 30. De plus, le bord avant de l'ergot 27 est arrondi, tandis que son bord arrière 32 est perpendiculaire à l'axe du poussoir.

Cet instrument comprend également une queue 33 en matière synthétique, de section cylindrique, comportant selon sa hauteur une pluralité de crans 34 annulaires et successifs formant une crémaillère dont la longueur est au moins égale à la course du piston 7 de la carpule 5. Chaque cran 34 possède une section décroissante de l'arrière vers l'avant, c'est-à-dire dans le sens de référence 8. La queue 33 est de section inférieure à la section intérieure du poussoir 22 et est destinée à être engagée dans celui-ci de l'arrière vers l'avant. L'extrémité supérieure de la queue 33 est équipée d'une collerette 35.

Le corps 2 d'une part, et le poussoir 22 d'autre part, possèdent chacun, à proximité de son extrémité inférieure, une série de dents respectivement 36 et 37. Les dents 36 d'une même série sont situées dans un même plan perpendiculaire à l'axe de l'instrument, et les dents 37 de l'autre série sont également disposées dans un même plan. Chaque dent 36, 37, est orientée radialement de l'extérieur vers l'intérieur et inclinée en direction de l'extrémité du corps contenant le réservoir 5. Chaque dent est adaptée à une pénétration entre deux crans successifs 34 de la crémaillère.

Il ressort de la structure, respectivement, du corps 2, du poussoir 22 et de la queue 33, que, lorsqu'une pression est exercée sur le poussoir 22 dans le sens de référence 8, il entraîne la queue, qui passe librement entre les dents 36 du corps 2. Au contraire, lorsque le poussoir est retiré, les dents 36 du corps 2 empêchent le retour de la queue 33, et les dents 37 du poussoir peuvent échapper aux crans 34 de la queue pour permettre le retour en arrière du poussoir.

L'utilisation de l'instrument, à partir de la configuration de la figure 1, est la suivante. L'instrument étant, tout d'abord, détaché du support sur lequel il est éventuellement fixé par une agrafe 40 que comporte le poussoir 22, il est procédé au réglage du volume de la dose de liquide à injecter. Par rotation du poussoir matérialisé par le déplacement circonférentiel du doigt 25, celui-ci est amené en regard du volume choisi et indiqué par une marque 26 sur le corps 2. Simultanément, l'ergot 27 se déplace en s'insérant puis en échappant aux différentes fentes 14 à 21 jusqu'à pénétrer dans la fente correspondant au volume retenu par l'utilisateur. En tirant le poussoir 22, l'utilisateur arme l'instrument, jusqu'à ce que l'ergot 27 vienne en butée contre l'extrémité haute de la fente 14-21 dans laquelle il se trouve. Lors de cette extraction, les dents 37 du poussoir glissent le long de la crémaillère 33.

L'utilisateur retire alors le capuchon 13, perfore le bouchon 9 avec une aiguille 10, si cela n'a pas encore été fait, avant de procéder à l'injection. Une pression sur le poussoir 22 se traduit par un déplacement dans le sens de référence 8 de ce poussoir, de la queue 33, et du piston 7, qui chasse le liquide 6 contenu dans le réservoir 5. Ce mouvement n'est pas perturbé par les dents 36 du corps qui laissent passer les crans de la queue compte tenu des inclinaisons respectives des dents 37 et des crans 34.

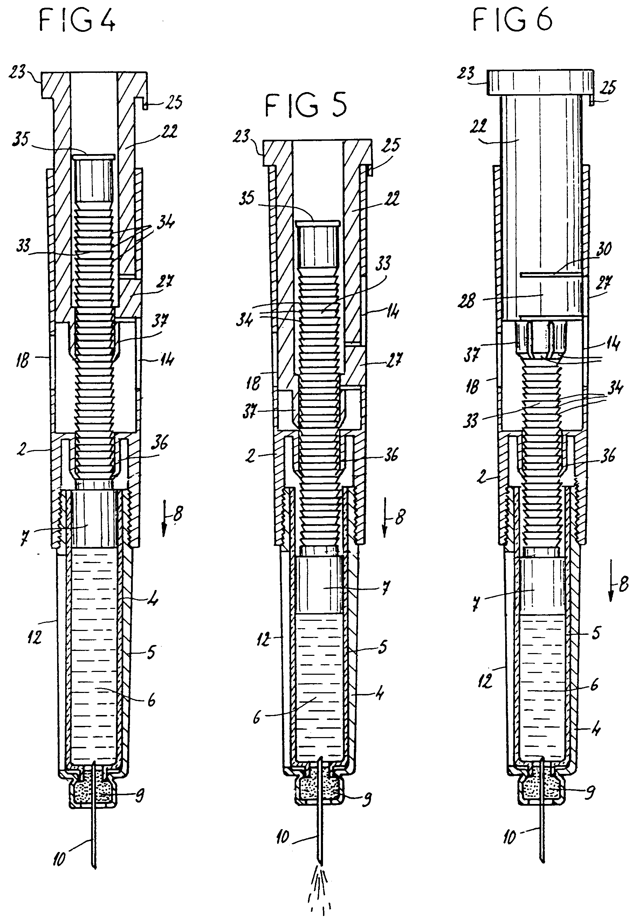

L'armement de l'instrument est représenté à la figure 4, tandis que la fin d'injection correspond à la position de figure 5. En fin d'injection, la collerette 23 du poussoir 2 vient en appui contre le bord supérieur du corps 2. Une fois l'injection terminée, le capuchon 13 est replacé sur l'extrémité avant de l'instrument.

La figure 6 représente l'instrument dans la même position qu'à la figure 5, le poussoir 22 étant vu en extérieur.

Il doit être noté que la collerette 35 de la queue 33 de l'instrument évite que l'on puisse réarmer correctement l'instrument lorsque le volume de liquide contenu dans le réservoir 5 est inférieur au volume de la dose devant être injectée. Par ailleurs, cette collerette évite qu'après démontage du réservoir, la queue 33 puisse être dégagée vers l'avant en vue d'une réutilisation de l'instrument.

Il ressort de ce qui précède que l'instrument selon l'invention est de conception simple, puisque comportant trois pièces en matière synthétique, à savoir un corps, un poussoir et une queue, qui réalisent les fonctions de réglage des doses, de visualisation des opérations, d'antiretour lors des mouvements relatifs de ces trois pièces. Il s'agit de pièces simples qui sont montées seulement par emboîtement, et dont la mise en oeuvre est également très simple, et peut être effectuée en toute sécurité même en cas d'urgence, ou par une personne physiquement diminuée.

## Revendications

1. Instrument doseur, notamment d'injection de liquide médicamenteux, pour dispenser plusieurs doses de liquide à partir d'une charge ou recharge comprenant :
- un corps tubulaire (2), destiné à être maintenu par l'utilisateur,
- un réservoir (5) contenant la charge ou recharge du liquide (6), rapporté sur ou intégré dans ledit corps, ce réservoir comprenant une paroi tubulaire dans l'axe du corps, un piston (7) formant joint d'étanchéité, fermant une extrémité de ladite paroi tubulaire, déplaçable vers l'autre extrémité, dans un sens (8) dit de référence, et un bouchon perforable (9) fermant l'autre extrémité, par lequel s'effectue le passage d'une dose du liquide,
- un poussoir tubulaire (22) monté coaxialement et libre en translation à l'intérieur du corps tubulaire (2), actionnable par l'utilisateur par une de ses extrémités qui dépasse du corps (2),
- une queue (33), formant crémaillère, montée à l'intérieur et dans l'axe du poussoir, comprenant une extrémité en appui contre le piston du réservoir,
- un premier moyen d'encliquetage (36) disposé sur le corps (2), échappant à la queue (33) dans le sens de référence (8), et bloquant la queue dans l'autre sens, et
- un deuxième moyen d'encliquetage (37) disposé sur le poussoir (22), se bloquant sur la queue (33) dans le sens de référence, et échappant à ladite queue dans l'autre sens,
la queue cylindrique (33) comportant une pluralité de crans (34) extérieurs successifs, ménagés selon l'axe du corps, sur une longueur au moins égale à la course du piston nécessaire pour vider le réservoir, coopérant à la fois avec le premier moyen d'encliquetage (36) et le deuxième moyen d'encliquetage (37), caractérisé en ce que les crans extérieurs (34) de la queue sont annulaires,
en ce que le corps présente plusieurs fentes axiales (14 - 21) de longueurs différentes les unes des autres, réparties sur la périphérie du corps (2),
et en ce que le poussoir (22) comporte un ergot (27) qui, faisant saillie de sa face extérieure, est destiné à être engagé dans l'une des fentes (14 - 21) du corps, dont la longueur correspond à la course choisie pour le poussoir.

2. Instrument selon la revendication 1, caractérisé en ce que la longueur de chaque fente axiale (14 - 21 )ménagée dans le corps (2) permet une course de l'ergot (27) égale à un nombre entier multiplié par la longueur d'un cran (34) de la crémaillère (33) ménagée sur la queue.

3. Instrument selon l'une quelconque des revendications 1 et 2, caractérisé en ce que l'ergot (27) faisant saillie de la face extérieure du poussoir est agencé pour s'effacer radialement lorsque le poussoir (22) est entraîné en rotation dans un sens à l'intérieur du corps, et/ou axialement lors de l'introduction du poussoir (22) à l'intérieur du corps (2).

4. Instrument selon la revendication 3, caractérisé en ce que l'ergot (27) faisant saillie de la face extérieure du poussoir (22) est constitué par une nervure axiale faisant saillie vers l'extérieur, ménagée à l'extrémité libre d'une languette (28) délimitée dans la paroi du poussoir par une fente axiale (29) et deux fentes circonférentielles (30) s'étendant sur une partie de la périphérie du poussoir.

5. Instrument selon la revendication 4, caractérisé en ce que le bord de la nervure formant ergot (27), situé en avant dans le sens d'introduction du poussoir dans le corps est arrondi, tandis que le bord arrière (32) de cette nervure est droit et perpendiculaire à l'axe du poussoir.

6. Instrument selon l'une quelconque des revendications 1 à 5, caractérisé en ce que l'extrémité du poussoir extérieure au corps et actionnable par l'utilisateur est équipée d'une collerette (23) destinée à venir prendre appui contre le bord arrière du corps (2), en fin de course d'injection.

7. Instrument selon la revendication 6, caractérisé en ce que les différentes fentes (14 à 21) ménagées dans le corps (2) présentent des extrémités avant situées dans un même plan perpendiculaire à l'axe du corps, la distance entre ce plan et l'extrémité arrière du corps (2) étant légèrement supérieure à la distance entre le bord avant de l'ergot (27) ménagé sur le poussoir (22) et la collerette (23) disposée à l'extrémité arrière de celui-ci.

8. Instrument selon la revendication 6, caractérisé en ce que la collerette (23) ménagée à l'extrémité postérieure du poussoir est équipée d'un doigt d'indexation (25), aligné avec l'ergot (27), et orienté en directon de l'avant du poussoir, ce doigt étant destiné, en fin de course avant du poussoir, à venir en recouvrement de la paroi extérieure du corps, en regard d'un marquage (26) que comporte le corps, dans l'alignement de chaque fente (14 - 21).

9. Instrument selon l'une quelconque des revendications 1 à 8, caractérisé en ce que la queue (33) est équipée, à son extrémité opposée à celle prenant appui sur le piston (7) d'une collerette (35) de diamètre extérieur inférieur au diamètre intérieur du poussoir, mais ne permettant pas le passage du moyen d'encliquetage du poussoir.

10. Instrument selon l'une quelconque des revendications 1 à 9, caractérisé en ce que chaque cran (34) possède une forme générale tronconique dont la petite section est tournée du côté de l'extrémité de la queue (33) prenant appui contre le piston (7).

11. Instrument selon la revendication 10, caractérisé en ce que les moyens d'encliquetage du corps et du poussoir sur la crémaillère de la queue sont constitués par deux séries de dents (36, 37) venant de moulage respectivement avec le corps et avec le poussoir, qui sont orientées chacune radialement de l'extérieur vers l'intérieur et sont inclinées en direction de l'extrémité du corps contenant le réservoir, les extrémités des dents d'une même série étant disposées dans un plan perpendiculaire à l'axe du corps (2), respectivement à l'axe du poussoir (22), et chaque dent étant adaptée à une pénétration entre deux crans successifs (34) de la crémaillère.

## Claims

1. Metering instrument, in particular for injecting medicinal liquids, for dispensing a number of measures of liquid from a supply or refill, comprising:
- a tubular body (2), which is to be held by user,
- a container (5) containing the supply or refill of the liquid (6), either added to or incorporated in said body, said container comprising a tubular wall axially aligned with the body, a piston (7) forming a sealed joint which closes off one end of said tubular wall and is displaceable towards the other end in a so-called reference direction (8), and a perforatable plug (9) closing off the other end, by means of which the passage of a measure of the liquid is effected,
- a tubular push rod (22) arranged coaxially and freely translatable within the tubular body (2), which is actuatable by the user by one of its ends projecting beyond the body (2),
- a shank (33) forming a serrated portion, arranged within and axially aligned with the push rod and comprising an end resting against the piston of the container,
- a first catching means (36) disposed on the body (2), moving away from the shank (33) in the reference direction (8) and locking the shank in the other direction, and
- a second catching means (37) disposed on the push rod (22), locking on the shank (33) in the reference direction and moving away from said shank in the other direction,
the cylindrical shank (33) comprising a plurality of successive external notches (34) arranged according to the axis of the body, over a length at least equal to the travel of the piston required to empty the container, cooperating both with the first catching means (36) and with the second catching means (37) characterised in that the external notches (34) of the shank are annular,
in that the body has a number of axial slots (14 - 21) of different lengths from one another, distributed over the periphery of the body (2),
and in that the push rod (22) comprises a tab (27) which, projecting from the external face of the push rod (22), is for engaging with one of the slots (14 - 21) in the body, and the length of which corresponds to the travel selected for the push rod.

2. Instrument according to claim 1, characterised in that the length of each axial slot (14 - 21) provided in the body (2) permits a travel of the tab (27) which is equal to a whole number multiplied by the length of a notch (34) in the serrated portion (33) provided on the shank.

3. Instrument according to any one of claims 1 and 2, characterised in that the tab (27) projecting from the external face of the push rod is arranged so as to move aside radially when the push rod (22) is rotated in a direction inside the body and/or axially on the introduction of the push rod (22) inside the body (2)

4. Instrument according to claim 3, characterised in that the tab (27) projecting from the external face of the push rod (22) is composed of an axial rib projecting towards the outside and disposed on the free end of a tongue (28) delimited in the wall of the push rod by an axial slot (29) and two circumferential slots (30) extending over part of the periphery of the push rod.

5. Instrument according to claim 4, characterised in that the edge of the rib forming a tab (27) which is situated to the front in the direction of introduction of the push rod into the body is rounded, while the rear edge (32) of said rib is straight and perpendicular to the axis of the push rod.

6. Instrument according to any one of claims 1 to 5, characterised in that the end of the push rod which is external to the body and actuatable by the user is fitted with a flange (23) intended to come to rest on the rear edge of the body (2) at the end of the injection travel.

7. Instrument according to claim 6, characterised in that the various slots (14 to 21) provided in the body (2) have front ends situated in one and the same plane perpendicular to the axis of the body, the distance between said plane and the rear end of the body (2) being slightly higher than the distance between the front edge of the tab (27) provided on the push rod (22) and the flange (23) disposed at the rear end of the latter.

8. Instrument according to claim 6, characterized in that the flange (23) provided at the posterior end of the push rod is fitted with an indexing finger (25) aligned with the tab (27) and pointing in the direction of the front of the push rod, said finger being intended, at the end of the forward travel of the push rod, to form an overlap with the external wall of the body, opposite a marking (26) which the body comprises, in alignment with each slot (14 - 21).

9. Instrument according to any one of claims 1 to 8, characterised in that the shank (33) is fitted, at its end opposite that resting on the piston (7), with a flange (35) of external diameter less than the internal diameter of the push rod, but not permitting passage of the catching means of the push rod.

10. Instrument according to any one of claims 1 to 9, characterized in that each notch (34) possesses a generally truncated form, the small section of which faces in the direction of the end of the shank (33) resting against the piston (7).

11. Instrument according to claim 10, characterized in that the catching means of the body and of the push rod on the serrated portion of the shank are composed of two series of teeth (36, 37) moulded integrally respectively with the body and with the push rod, which are each oriented radially from the outside to the inside and are inclined in the direction of the end of the body containing the container, the ends of the teeth of one and the same series being disposed in a plane perpendicular to the axis of the body (2), respectively to the axis of the push rod (22), and each being adapted to entering between two successive notches (34) in the serrated portion.

## Patentansprüche

1. Dosierinstrument, insbesondere zum Injizieren einer Medikamentenflüssigkeit, zur Abgabe mehrerer Flüssigkeitsportionen aus einer Füllung oder Nachfüllung, umfassend:
- einen rohrförmigen Körper (2), der dazu bestimmt ist, vom Benutzer gehalten zu werden,
- ein die Flüssigkeitsfüllung oder -nachfüllung (6) enthaltendes, an dem Körper angebrachtes oder in diesen integriertes Reservoir (5), wobei dieses Reservoir eine rohrförmige Wandung in Achsrichtung des Körpers, einen eine Dichtung bildenden und ein Ende der rohrförmigen Wandung verschließenden Kolben (7), welcher in einer als Bezugsrichtung bezeichneten Richtung (8) zu dem anderen Ende hin verlagerbar ist, sowie einen das andere Ende verschließenden, durchstoßbaren Stopfen (9) umfaßt, durch welchen der Durchtritt einer Flüssigkeitsportion erfolgt,
- einen koaxial und längsbeweglich im Innern des rohrförmigen Körpers (2) angeordneten rohrförmigen Drücker (22), welcher mit einem aus dem Körper (2) herausragenden seiner Enden seitens des Benutzers betätigbar ist,
- ein eine Verzahnung bildendes, im Innern des Drückers und in dessen Achsrichtung angeordnetes Stangenelement (33) mit einem an dem Kolben des Reservoirs anliegenden Ende,
- ein erstes, an dem Körper (2) angeordnetes Rastmittel (36), welches das Stangenelement (33) in Bezugsrichtung (8) freigibt und das Stangenelement in der anderen Richtung blockiert, sowie
- ein zweites, an dem Drücker (22) angeordnetes Rastmittel (37), welches in Bezugsrichtung an dem Stangenelement (33) blockiert und in der anderen Richtung das Stangenelement freigibt,
wobei das zylindrische Stangenelement (33) eine Mehrzahl aufeinanderfolgender Außenvertiefungen (34) aufweist, welche in Achsrichtung des Körpers auf einer Länge ausgebildet sind, die zumindest gleich dem zum Leeren des Reservoirs nötigen Kolbenhub ist, und sowohl mit dem ersten Rastmittel (36) als auch dem zweiten Rastmittel (37) zusammenwirken,
**dadurch gekennzeichnet,** daß die Außenvertiefungen (34) des Stangenelements ringförmig sind, daß der Körper mehrere über den Umfang des Körpers (2) verteilte axiale Schlitze (14 - 21) mit voneinander verschiedenen Längen aufweist und daß der Drücker (22) eine von seiner Außenseite vorstehende Nase (27) aufweist, welche dazu bestimmt ist, in einen der Schlitze (14 - 21) des Körpers einzugreifen, dessen Länge dem für den Drücker gewählten Hub entspricht.

2. Instrument nach Anspruch 1, **dadurch gekennzeichnet,** daß die Länge jedes in dem Körper (2) ausgebildeten axialen Schlitzes (14 - 21) einen Hub der Nase (27) erlaubt, der gleich einem ganzzahligen Vielfachen der Länge einer Vertiefung (34) der an dem Stangenelement ausgebildeten Verzahnung (33) ist.

3. Instrument nach einem der Ansprüche 1 und 2, **dadurch gekennzeichnet,** daß die von der Außenseite des Drückers vorstehende Nase (27) dazu ausgebildet ist, sich bei Drehung des Drückers (22) radial in Richtung zum Körperinnern hin zurückzuziehen und/oder axial bei Einführung des Drückers (22) in das Innere des Körpers (2).

4. Instrument nach Anspruch 3, **dadurch gekennzeichnet,** daß die von der Außenseite des Drückers (22) vorstehende Nase (27) von einer nach außen hin vorstehenden axialen Rippe gebildet ist, welche am freien Ende einer Lasche (28) ausgebildet ist, die in der Wandung des Drückers durch einen Axialschlitz (29) und zwei sich über einen Teil des Drückerumfangs erstreckende Umfangsschlitze (30) begrenzt ist.

5. Instrument nach Anspruch 4, **dadurch gekennzeichnet,** daß der in Einführrichtung des Drückers in den Körper vorne liegende Rand der die Nase (27) bildenden Rippe abgerundet ist, während der hintere Rand (32) dieser Rippe gerade und orthogonal zur Drückerachse ist.

6. Instrument nach einem der Ansprüche 1 bis 5, **dadurch gekennzeichnet,** daß das außerhalb des Körpers befindliche und durch den Benutzer betätigbare Ende des Drückers mit einem Bund (23) versehen ist, welcher dazu bestimmt ist, am Ende des Injektionshubs zur Anlage an dem hinteren Rand des Körpers (2) zu gelangen.

7. Instrument nach Anspruch 6, **dadurch gekennzeichnet,** daß die in dem Körper (2) ausgebildeten verschiedenen Schlitze (14 bis 21) vordere Enden besitzen, welche in ein und derselben zur Körperachse orthogonalen Ebene liegen, wobei die Entfernung zwischen dieser Ebene und dem hinteren Ende des Körpers (2) etwas größer als die Entfernung zwischen dem vorderen Rand der an dem Drücker (22) ausgebildeten Nase (27) und dem am hinteren Ende desselben angeordneten Bund (23) ist.

8. Instrument nach Anspruch 6, **dadurch gekennzeichnet,** daß der am hinteren Ende des Drückers ausgebildete Bund (23) mit einem zu der Nase (27) ausgerichteten und in Richtung des Drückervorderteils orientierten Indexfinger (25) versehen ist, wobei dieser Finger dazu bestimmt ist, am Ende des Vorwärtshubs des Drückers in Überdeckung mit der Außenwandung des Körpers zu gelangen, und zwar in Gegenüberlage zu einer Markierung (26), welche der Körper in Ausrichtung zu dem jeweiligen Schlitz (14 - 21) trägt.

9. Instrument nach einem der Ansprüche 1 bis 8, **dadurch gekennzeichnet,** daß das Stangenelement (33) an seinem Ende, das dem an dem Kolben (7) anliegenden entgegengesetzt ist, mit einem Bund (35) versehen ist, dessen Außendurchmesser kleiner als der Innendurchmesser des Drückers ist, jedoch nicht die Vorbeibewegung des Rastmittels des Drückers erlaubt.

10. Instrument nach einem der Ansprüche 1 bis 9, **dadurch gekennzeichnet,** daß jede Vertiefung (34) eine kegelstumpfartige Grundform besitzt, deren kleiner Querschnitt der Seite des an dem Kolben (7) anliegenden Endes des Stangenelements (33) zugewandt ist.

11. Instrument nach Anspruch 10, **dadurch gekennzeichnet,** daß die Rastmittel des Körpers und des Drückers an der Verzahnung des Stangenelements von zwei zusammen mit dem Körper bzw. dem Drücker ausgeformten Zahnreihen (36, 37) gebildet sind, welche jeweils radial von außen nach innen orientiert sind und in Richtung zu dem das Reservoir aufweisenden Ende des Körpers hin geneigt sind, wobei die Enden der Zähne einer Reihe in einer zur Achse des Körpers (2) bzw. zur Achse des Drückers (22) orthogonalen Ebene angeordnet sind und jeder Zahn dazu ausgebildet ist, zwischen zwei aufeinanderfolgende Vertiefungen (34) der Verzahnung einzugreifen.
